# EUROPEAN PATENT SPECIFICATION

(11) **EP 0 719 556 B1**
(45) Date of publication and mention of the grant of the patent: **25.10.2000**
(21) Application number: 95120021.1
(22) Date of filing: 19.12.1995
(51) Int. Cl.: A61K 31/415

(54) **Use of (2S,4S)-6-fluoro-2',5'-dioxospiro[chroman-4,4'-imidazoline]-2-carboxamide for treating diabetic keratopathy**
Verwendung von (2S,4S)-6-fluoro-2',5'-dioxospiro[chroman-4,4'-imidazoline]-2-carboxamid zur Behandlung von Keratopathie
Utilisation de (2S,4S)-6-fluoro-2',5'-dioxospiro[chroman-4,4'-imidazoline]-2-carboxamide pour le traitement de la kératopathie

(30) Priority: 28.12.1994 JP 32759094
(43) Date of publication of application: 03.07.1996
(73) Proprietor: SANWA KAGAKU KENKYUSHO CO., LTD., Higashi-ku Nagoya-shi Aichi-ken (JP)
(72) Inventor: Sawai, Kiichi, c/o Sanwa Kagaku Kenkyusho Co., Ltd, Nagoya-shi, Aichi (JP); Ban, Masatoshi, c/o Sanwa Kagaku Kenkyusho Co, Ltd, Nagoya-shi, Aichi (JP); Sato,Makoto, c/o Sanwa Kagaku Kenkyusho Co, Ltd, Nagoya-shi, Aichi (JP); Hibi, Chihiro, c/o Sanwa Kagaku Kenkyusho Co, Ltd, Nagoya-shi, Aichi (JP); Mizuno, Kuniharu, c/o Sanwa Kagaku Kenkyusho Co., Nagoya-shi, Aichi (JP)
(74) Representative: Weisert, Annekäte, Dipl.-Ing. Dr.-Ing.

(56) References cited:
- EP-A- 0 264 586
- EP-A- 0 437 025
- EP-A- 0 485 219
- US-A- 4 900 739
- DATABASE CHEMABS CHEMICAL ABSTRACTS SERVICE, COLUMBUS, OHIO, US AN: 105:223857, Y. TAKAHASHI ET AL: "The role of aldose reductase in diabetic corneal epitheliopathy. 2. The corneal epithelium of galactosemic rats " XP002020272
- JOURNAL OF CHEMICAL EDUCATION, vol. 63, no. 3, March 1986, USA, pages 243-245, XP000195910 J. J. ARES ET AL: "Treatment of Diabetic Complications with Aldose Reductase Inhibitors"
- PREVENTIVE MEDICINE, vol. 23, no. 5, September 1994, USA, pages 717-721, XP000195904 P. F. KADOR ET AL: "Amelioration of Diabetes-like retinal Changes in Galactose-Fed Dogs"
- DATABASE STN, DRUGUPDATES, STN no. AN: 93:709, : "",

## Description

This invention relates to the use of (2S,4S)-6-fluoro-2',5'-dioxospiro[chroman-4,4'-imidazoline]-2-carboxamide for the manufacture of a medicament for treating diabetic keratopathy. More particularly, it relates to the use wherein said medicament is in the form of, for example, eye drops for use in the treatment of diabetic corneal epitheliopathy, diabetic corneal imperception and diabetic corneal endotheliopathy.

(2S,4S)-6-Fluoro-2',5'-dioxospiro[chroman-4,4'-imidazoline]-2-carboxamide, which is a compound found out by the company to which the present inventors belong, exhibits therapeutic effects on diabetic neuropathy, an antiulcer effect and a pharmacological action on circulatory organs. Because of the high safety over a prolonged administration term, it is now under clinical examination as a medicine for oral use (JP-B-3-72227 corresponding to EP-B-264586 and U.S. Patent No. 4,985,573, JP-A-3-215435 corresponding to U.S. Patent No. 5,155,125, JP-A-3-106885 corresponding to U.S. Patent No. 5,164,391, etc.; the term "JP-B" as used herein means an "examined Japanese patent publication", and the term "JP-A" as used herein means an "unexamined published Japanese patent application").

Chemical Abstracts, AN 105:223857 (Nippon Ganka Gakkai Zasshi (1986)), 90(2), 336-340, Y. Takahashi et al. discloses the topical administration of the aldose reductase inhibitor M-79175 into the corneal epithelium of galactosemic rats.

US-A-4 900 739 discloses spirosuccinimides for treating diabetic complications such as keratopathy.

EP-A-0 485 219 relates to medicaments containing spiro-3-heteroazolidine compounds for preventing and treating circulatory organ diseases.

Diabetic keratopathy is a disease accompanied by various abnormalities in the corneal epithelium, perception (Schwann cells) and endothelial cells. When the cornea undergoes severe disintegration, vesicular keratopathy occurs and the visual function is seriously damaged, thus frequently becoming intractable. However, the occurrence mechanism of diabetic keratopathy has not been clarified and no effective treating agent therefor has been developed so far.

Although corneal endothelial cells have important functions, they have no or little regenerative power. However, a diabetic patient suffers from simple diabetic retinopathy about 10 years after the outbreak of diabetes. Subsequently, the disease can proceed to, proliferating retinopathy, retinal detachment and loss of sight in some cases. Known methods for preventing the advance of the disease include photocoagulation, operation on corpus vitreum and intraocular lens replacement for cataracta. It is considered, however, that a strong stress induced by such a treatment easily affects the corneal endothelial cells to thereby induce keratopathy.

When the endothelial cells of a diabetic are observed with a speculative microscope, it is found that the fragility of the cornea induces morphological abnormalities (irregular size, deformation, etc.), which already suggests the occurrence of diabetic corneal endothelial disorders, even though the disease does not advance into the stage of keratopathy.

Although the exact number of patients with diabetic keratopathy has not been reported so far, Shimizu et al., Department of Ophthalmology, Kitasato University have reported that diabetic patients suffer from corneal damage at a higher ratio of 56% than that of normal persons (17%) and that the corneal tissue of a diabetic patient is more vulnerable than that of a normal person. At the present stage, the number of potential diabetics amounts to 6,000,000 and is considered to be increasing. Since diabetes causes serious problems including an increase in the medical expenses therefor and damage to the quality of life (QOL) of patients, it has been urgently required to develop a medicine efficacious against this disease.

The present inventors have found out that (2S,4S)-6-fluoro-2',5'-dioxospiro[chroman-4,4'-imidazoline]-2-carboxamide is effective in improving diabetic keratopathy for which no efficacious therapy has been established so far. Accordingly, the present invention provides a medicine which is highly useful from the viewpoints of medical expenses, welfare and quality of life of patients.

(2S,4S)-6-Fluoro-2',5'-dioxospiro[chroman-4,4'-imidazoline]-2-carboxamide used in the present invention as an active ingredient may be synthesized in accordance with the known method (cf. EP-B-264586 corresponding to U.S. Patent No. 4,985,573, herein incorporated by reference).

The agent used for treating diabetic keratopathy of the present invention may be processed into a dosage form of, for example, oral preparations such as tablets, capsules, powders and granules or parenteral preparations such as eye drops, injections and suppositories by using carriers, excipients, or the like commonly used in the art in accordance with the conventional pharmaceutical techniques (cf. *Nihon Yakkyokuho Seizaisousoku* (General rules for preparations in Pharmacopeia of Japan (JP), herein incorporated by reference). The dose varies depending on the conditions and age of a patient, the administration route, the dosage form, etc. In the case of eye drops, the content of the active ingredient is controlled to 0.01 to 1% and the eye drops are dropped to eyes one to several times per day. In the case of oral administration, the doze per an adult is from 0.125 to 100 mg, preferably from 0.5 to 2 mg, in terms of the active ingredient of the preparation and it is administered in one to several portions per day, thus achieving the object.

Examples of the subject to be treated in the present invention includes mammals, especially, human.

To further illustrate the present invention in greater detail, the following Test Examples and Preparation Examples will be given.

Unless otherwise indicated, all parts, percents, ratios and the like are by weight.

### TEST EXAMPLE 1

### Test method:

The experiment was carried out in accordance with the method of Akagi et al. (*Nihon Ganka Kiyo* (Bulletin of Japanese Ophthalmology), 37, 809, 1986).

Sprauge Dawley rats weighing 50 g and aged 3 weeks were divided into the following 3 groups each having 15 mice. (1) A control group which was fed with a normal feed for laboratory use (normal control group). (2) A group which was fed with a feed containing 50% of galactose (galactose group). (3) A group which were fed with a feed containing 50% of galactose together with 3 mg/kg forced oral administration of (2S,4S)-6-fluoro-2',5'-dioxospiro[chroman-4,4'-imidazoline]-2-carboxamide (hereinafter referred to simply as "compound A") (compound A/galactose group). The rats of each group were fed for about 6 weeks. The freeze disruption of the corneal endothelial cells was performed by applying a stainless bar (diameter: 1.5 mm), which had been cooled to -70°C with acetone/dry ice, to the center of the cornea of each animal for 20 seconds to thereby intracorneally disrupt the endothelial cells. After the completion of the freeze disruption, the cornea was extracted under euthanasia with nembutal and observed in the following manner with the passage of time.

Light microscopic observation: The cornea was fixed with a 0.1 M phosphate buffer solution (pH 7.4) containing 1% of paraformaldehyde and 1 % of glutaraldehyde and re-fixed with 1% osmium tetraoxide. Then, it was dehydrated with alcohol and embedded into Epon resin by a conventional method. Prior to the light microscopic observation, sections were stained with Toluidine Blue.

Observation of extended sample: Unfixed cornea was immersed in a 60 mM 2Na·EDTA buffer solution and fixed with methanol. Then, the endothelial cell layer alone was peeled from Descemet's membrane and attached to a slide glass. Each sample was stained with Toluidine Blue and then observed under a light microscope.

### Results:

5 days after freeze disruption: In the normal control group, a 2- or 3-layered arcuate stratified region was observed in the frozen site and the endothelial cells had been repaired so long as observed under the light microscope. In the galactose group, on the other hand, several large round bulgings were observed in the frozen site. Each bulging contained denatured cells therein. Although the compound A/galactose group sometimes showed bulgings too, they were smaller in size. The extended sample showed a site crowded with a number of nuclei at the center of the cornea.

7 days after freeze disruption: The normal control group and the compound A/galactose group showed no stratified region any longer at this stage. Namely, the samples of these groups had been completely flattened and normalized. On the other hand, more than a half of the samples of the galactose group still showed bulgings even at this stage. Compared with the images obtained on the day 5, the stratified regions were reduced in height and the endothelial cells were composed of not round but long and narrow cytoplasm. Also, the extended sample showed a site crowded with a number of stratifying nuclei over a wide range.

### TEST EXAMPLE 2

### Test method:

Based on the results of the Test Example 1, further observation test was carried out to confirm the clear utility of the present invention.

The test was carried out by the similar method as in Example 1. After 5 days from the freeze disruption, the cornea was extracted from (1) the normal control group, (2) the galactose group, and (3) the compound A/galactose group.

The left eye cornea of the rat was embedded into paraffine by a conventional method. The sample was sliced and the resulting sections were stained with Hematoxylin-Eosin and observed with a light microscope.

### Results:

Light microscopic observation: The stratified region and bulgings of the corneal endothelial cells at the frozen region are considered to represent a repairing process of damages in the corneal endothelial cells. Five days after the freeze disruption, such phenomena were observed in 25% of the eyeballs in the normal group. On the other hand, such phenomena were observed in 100% of eyeballs in the galactose group, and the difference between these group is statically significant (P < 0.05). Accordingly, the repair of damages in the corneal endothelial cells of the galactose group was significantly delayed. In the compound A/galactose group, the stratified region and bulgings of the corneal endothelial cells were observed in 22% of eyeballs, which was significantly (p < 0.01) normalized in comparison with the galactose group.

| Effect on delayed repair of damages in the corneal endothelial cells with freeze disruption in the galactosemia mice (observation after 5 days from the freeze disruption) | | | | |
|---|---|---|---|---|
| Treatment | | Number of observed corneas | Number of corneas showing delayed repair of damages | (%) |
| (1) | Normal | 8 | 2 | (25) |
| (2) | Galactose | 8 | 8 | (100) |
| (3) | compound A/galactose | 9 | 2 | (22) |

From these results, it was statically clarified that the administration of compound A improves the delayed repair of damages in the corneal endothelial cells.

As described above, the administration of compound A improves the delayed repair of damages in the corneal endothelial cells in rats with galactosemia, namely, the most convenient animal model of diabetes. Thus, it is strongly suggested that compound A is clinically usable in the treatment of diabetic keratopathy.

### PREPARATION EXAMPLE 1

Compound A: (2S,4S)-6-fluoro-2',5'-dioxospiro[chroman-4,4'-imidazoline]-2-carboxamide.

| Formula A | |
|---|---|
| per 100 ml | |
| | |
| Compound A | 0.10 g |
| Sodium monohydrogenphosphate | 0.76 g |
| Sodium dihydrogenphosphate | 0.16 g |
| Sodium chloride | 0.42 g |
| Benzalkonium chloride | 0.01 g |
| Sterile purified water | q.s. |

### Production method

To 80 ml of sterile purified water are added sodium dihydrogenphosphate, sodium monohydrogenphosphate and benzalkonium and dissolved therein. Then, the compound A is added thereto.

After dissolving the compound A, sterile purified water is added so as to adjust the total volume of the solution to 100 ml. The obtained product is used as eye drops.

| Formula B | |
|---|---|
| per 100 ml | |
| | |
| Compound A | 0.10 g |
| Sodium monohydrogenphosphate | 0.76 g |
| Sodium dihydrogenphosphate | 0.16 g |
| Sodium chloride | 0.40 g |
| Benzalkonium chloride | 0.01 g |
| Sucrose | 0.05 g |
| Sterile purified water | q.s. |

### Production method

The procedure of Formula A is followed to thereby give eye drops.

### PREPARATION EXAMPLE 2

| Formula C | |
|---|---|
| per 100 ml | |
| | |
| Fat emulsion | 50.0 g |
| Sodium monohydrogenphosphate | 0.76 g |
| Sodium dihydrogenphosphate | 0.16 g |
| Sodium chloride | 0.40 g |
| Benzalkonium chloride | 0.01 g |
| Sucrose | 0.05 g |
| Sterile purified water | q.s. |

### Production method

The procedure of Formula A is followed to thereby give eye drops.

### PREPARATION EXAMPLE 3

The electric charge controlling agents as listed in the following Table 1 (0.4 g) are respectively mixed with 1.6 g of purified yolk lecithin and 8.0 g of the oily components as listed in the following Table 2 in 50 ml of a mixture of chloroform/methanol (5/1, v/v). After dissolving, 0.1 to 1.0 g of the compound A is added thereto and the solvent is completely removed. Then, 90 g of sterile purified water is added thereto and the obtained mixture is emulsified under elevated pressure (1,500 kg/cm²) with the use of a microfluidizer. Thus, a 10% (w/w) fat emulsion is obtained.

**TABLE 1**

| Electric charge controlling agent |
|---|
| Dimyristoylphosphatidylglycerol |
| Dimyristoylphosphatidic acid |
| Phosphatidylinositol |
| Phosphatidylserine |
| Oleic acid |
| Sodium caprate |

**TABLE 2**

| Oily component |
|---|
| Soybean oil |
| Medium-chain fatty acid triglyceride |
| Tocopherol acetate |
| Squalane |

### PREPARATION EXAMPLE 4

| Formula | |
|---|---|
| Compound A | 2 mg |
| Sucrose | 25 mg |
| Crystalline cellulose | 30 mg |
| Lactose | q.s. |
| | 180 mg |

### Production method

The compound A and sucrose are kneaded with the use of ethanol/water (1/1, w/w). After drying, crystalline cellulose and lactose are added thereto. Next, a lubricant commonly employed in the art is added thereto. The obtained mixture is processed into oral preparations such as tablets, capsules, granules, etc. by a conventional method.

### PREPARATION EXAMPLE 5

| Formula | |
|---|---|
| Compound A | 1 mg |
| Sucrose | 10 mg |
| Crystalline cellulose | 30 mg |
| Lactose | q.s. |
| | 180 mg |

### Production method

The procedure of Pharmaceutical Example 4 is followed to thereby give oral preparations.

The mechanism of diabetic keratopathy occurrence has not been clarified and thus no effective treating agent therefor has been developed so far. It is a disease accompanied by various abnormalities in the corneal epithelium, perception (Schwann cells) and endothelial cells. When the cornea undergoes severe disintegration, vesicular keratopathy occurs and seriously damages the visual function. Thus, it frequently becomes intractable. The present invention provides an agent for treating diabetic keratopathy which comprises (2S,4S)-6-fluoro-2',5'-dioxospiro[chroman-4,4'-imidazoline]-2-carboxamide as an active ingredient. It is in the form of, in particular, oral preparations or eye drops and efficacious against diabetic corneal epitheliopathy, diabetic corneal imperception and diabetic corneal endotheliopathy.

## Claims

1. Use of (2S,4S)-6-fluoro-2',5'-dioxospiro[chroman-4,4'-imidazoline]-2-carboxamide for the manufacture of a medicament for treating diabetic keratopathy.

2. The use as claimed in claim 1, wherein said diabetic keratopathy is diabetic corneal epitheliopathy, diabetic corneal imperception or diabetic corneal endotheliopathy.

3. The use as claimed in claim 1, wherein said medicament is in the form of eye drops or oral preparations.

## Patentansprüche

1. Verwendung von (2S,4S)-6-Fluor-2',5'-dioxospiro[chroman-4,4'-imidazolin]-2-carboxamid zur Herstellung eines Medikaments für die Behandlung von diabetischer Keratopathie.

2. Verwendung nach Anspruch 1, wobei die diabetische Keratopathie eine diabetische korneale Epitheliopathie, eine diabetische korneale Wahrnehmungsstörung oder eine diabetische korneale Endotheliopathie ist.

3. Verwendung nach Anspruch 1, wobei das Medikament in Form von Augentropfen oder oralen Präparaten vorliegt.

## Revendications

1. Utilisation du (2S,4S)-6-fluoro-2',5'-dioxospiro[chroman-4,4'-imidazoline]-2-carboxamide pour la fabrication d'un médicament pour le traitement de la kératopathie diabétique.

2. Utilisation selon la revendication 1, dans laquelle ladite kératopathie diabétique est une épithéliopathie de la cornée chez un diabétique, un trouble cornéen de la perception chez un diabétique et une endothéliopathie de la cornée chez un diabétique.

3. Utilisation selon la revendication 1, dans laquelle ledit médicament est sous la forme de collyres ou de préparations orales.
